(19) Europäisches Patentamt

European Patent Office

Office européen des brevets

(11) Publication number: 0 353 342 A1

## (12) EUROPEAN PATENT APPLICATION

(21) Application number: 88116022.0

(22) Date of filing: 28.09.88

(51) Int. Cl.⁴: C12N 9/24 , //(C12N9/24, C12R1:645)

The microorganism(s) has (have) been deposited with the American Type Culture Collection under number(s) 53812.

(30) Priority: 27.07.88 US 224590

(43) Date of publication of application:
07.02.90 Bulletin 90/06

(84) Designated Contracting States:
AT BE CH DE ES FR GB IT LI NL SE

(71) Applicant: Iowa State University Research Foundation, Inc.
315 Beardshear
Ames, Iowa 50011(US)

(72) Inventor: Vartak, Hari Gopal
1161/6 Shivajinagar
Gharpure Colony Pune 411005(IN)
Inventor: Bastawde, Kulbhushan Balwant
F-2(G) NCL Colony
Pasham Road Pune 41108(IN)

(74) Representative: Patentanwälte Grünecker, Kinkeldey, Stockmair & Partner
Maximilianstrasse 58
D-8000 München 22(DE)

(54) Low molecular weight xylanase glycoprotein.

(57) A low molecular weight endo-xylanase has been isolated from an actinomycete strain of Chainia. The purified enzyme which has been partially sequenced is a glycoprotein with a molecular weight between 5,000 and 9,000, estimated at 6,000 ± 1,000. This small molecule xylanase has specific activity for xylan, selectively converting it to oligosaccharides, the enzyme having no significant activity for cellulose.

FIG. I

# LOW MOLECULAR WEIGHT XYLANASE GLYCOPROTEIN

The field of this invention is xylanase enzymes which act on plant xylan, such as xylanases produced by microorganisms.

Endo-xylanases (1,4-$\beta$-D-xylan xylanohydrolase) which hydrolyze mainly interior (1→4)-$\beta$-D-xylosyl bonds in the plant hemicellulose xylan, have been isolated from fungi, bacteria, plants, and invertebrates. But there has been relatively little study of endo-xylanases from actinomycetes despite the major scientific and commercial importance of this bacterial family.

In the earliest known reference, Iizuka and Kawaminami (1965) reported an endo-xylanase from Streptomyces xylophagus. It initially produced xylobiose and larger xylo-oligosaccharides from xylan, but yielded finally xylobiose and D-xylose. Later, in two reports, Kusakabe et al. (1977) measured the optimum activity and stability of a similar enzyme from Streptomyces. This endo-xylanase was found to have a molecular weight of approximately 40,500. It produced D-xylose and xylobiose from xylan after originally forming xylooligosaccharides of intermediate length, and evidently had some xylosyltransferase activity, as it yielded xylobiose but no D-xylose from xylotriose. [Kusakabe, Kawaguchi et al. (1977); and Kusakabe, Yasui et al. (1977).] Nakajima et al. (1984) has also described a similar Streptomyces endo-xylanase of around 43,000 daltons molecular weight.

Researchers in the National Chemical Laboratory at Pune, India have reported the isolation of enzyme-producing strain of an actinomycete strain of the genus Chainia. [Srinivasan et al. (1983).] This strain was assigned the code identification "NCL 82-5-1" and has been studied within the National Chemical Laboratory (hereinafter NCL). [Srinivasan, et al. (1984).] Chainia strain NCL 82-5-1 has not been available outside of NCL, and has been maintained by NCL on a non-public basis.

This application is based on the discovery of a novel endo-xylanase which can be obtained by fermentation of strain NCL-82-5-1 on a medium containing a xylan source, separation of the fermentation liquid from the solids, and subjecting the liquid to a series of chromatographic separations to isolate and purify the endo-xylanase of this invention, which is referred to herein as Xylanase II. This enzyme is a glycoprotein of low molecular weight. As far as is known, Xylanase II has the smallest molecular weight of any xylanase yet reported. Xylanase II has been obtained in substantially pure form. Its absolute molecular weight as expressed in daltons is below 9,000 and above 5,000. From available data, the estimated molecular weight in daltons is 6000 ± 1000. Other identifying criteria have been determined. The approximate amino acid content and number of amino acids are known, and most of the amino acid backbone of the glycosylated protein has been sequenced. In fact, an exact sequence has been confirmed by repeated analysis for the major portion of the protein.

Xylanase II has important properties for use in processing of wood fibers. It has specific enzymatic activity for xylan with no appreciable enzymatic activity for cellulose. Its small molecular size permits Xylanase II to penetrate wood fibers so that it can perform a selective degradation of xylan. Xylanase II converts xylan to xylooligosaccharides. Xylanase II can also be used to act on xylan in other substrates, since it does not have an appreciable enzymatic action against cellulose derivatives or other oligosaccharides such as starch.

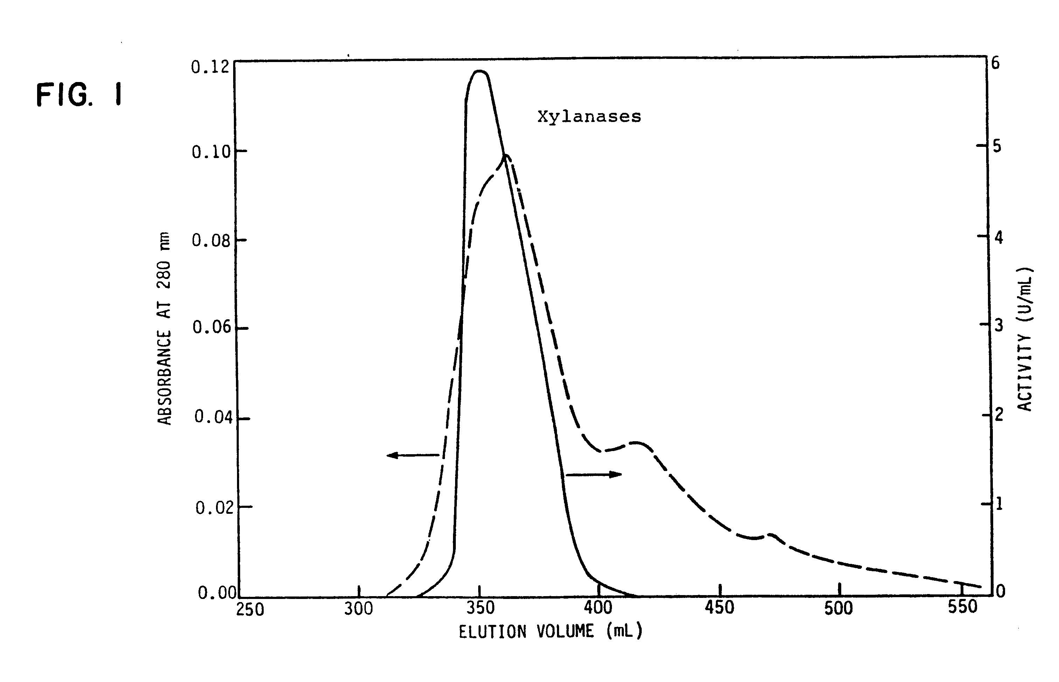

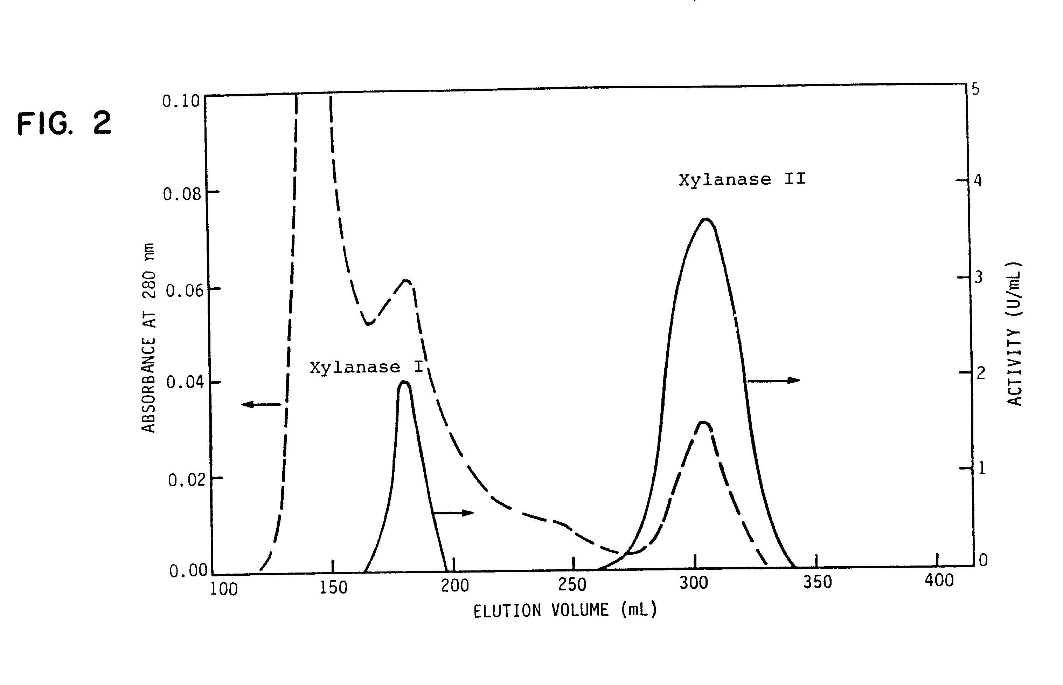

The accompanying drawings are plots of chromatographic separations of the xylanases. Fig. 1 shows the initial separation of the endo-xylanase activity from the fermentation medium, the peak shown including both the large molecule xylanases. Fig. 2 represents a further resolution of this endo-xylanase activity into two fractions. the first peak being a large molecular xylanase (Xylanase I), and the second peak the small molecule xylanase (Xylanase II) of this invention.

This invention relates to a novel endo-xylanase which is a substantially pure glycoprotein having a molecular weight greater than 5000 and less than 9000 (as expressed in daltons). This enzyme, referred to as Xylanase II. is characterized by specific enzymatic activity for xylan with no appreciable enzymatic activity for cellulose. By the method subsequently described, Xylanase II can be purified to essential homogeneity. The Xylanase II is further characterized by a 24 amino acid sequence as subsequently described.

The preferred method for preparing Xylanase II involves the use of the Chainia strain NCL-82-5-1, which will be made permanently available to the public. Utilization of strain NCL-82-5-1 for producing Xylanase II involves the following general procedures, as illustrated in detail by the Experimental Examples.

NCL-82-5-1 is a sclerotia-forming actinomycete of the genus Chainia which secretes extracellular glucose isomerase, xylose isomerases, and xylanases when grown in an appropriate media. For example. a wheat bran-yeast extract medium can be employed to produce the extracellular endo-xylanases, which include the large molecule, Xylanase I, having an apparent relative molecular weight of the order of 25,000.

and the small molecule Xylanase II, of this invention.

Fermentation media which can be employed for culturing NCL 82-5-1 are described in Srinivasan et al (1983) and Srinivasan et al (1984). For preparing inoculation cultures, the Chainia strain may be cultured and/or maintained on potato dextrose agar and malt extract glucose peptone agar. Periodic subculturing is desirable to maintain viability. The aqueous fermentation media for the xylanase production should contain a xylan source. For example, wheat bran and similar xylan containing vegetable sources can be used. More specifically, a suitable medium may be prepared from 5% wheat bran and 1% yeast extract. The starting pH may be around 5.8, and the fermentation may be carried out at temperatures of around 28°C. Fermentation is continued until the extracellular xylanases are liberated. Fermentation times of from 90 to 120 hours are suitable, but shorter or longer times can be used. The progress of the fermentation can be followed by withdrawing samples and testing for the extracellular xylanase activity.

On completion of the fermentation, the Chainia cells are separated from the aqueous fermentation medium, viz. by centrifugation, filtration, or other procedure. The cell-free medium containing xylanases is further processed to recover the Xylanase II endo-xylanase. In a preferred procedure, ethanol is added to the clarified medium to precipitate the xylanases, including the large molecule Xylanase I and the desired small molecule Xylanase II. The precipitated xylanases are redissolved and subjected to a series of chromatographic separations. The Xylanase II is thereby prepared in a highly purified form.

## EXPERIMENTAL EXAMPLES

## Materials and Methods

Cultural conditions: The growth and fermentation medium included wheat bran (5.0 g) and yeast extract (1.0 g) in 100 ml distilled water. Medium was autoclaved at 121°C for 40 min. Seven-day-old culture, grown on 2% potato dextrose agar slant was used to inoclate the inoculum flasks. The flasks were further incubated for 72 h and were used as an inoculum. The experimental flasks were inoculated with the above inoculum and incubated at 30°C on a rotary shaker (200 rpm) for 120 h. Culture filtrate was collected after centrifugation (700 x g) as the clear supernatant.

Xylanase assay: Xylanase activity was measured by estimating reducing sugars produced, as xylose equivalents, by 3,5-dinitrosalicylic acid reagent (Miller, 1959) as well as by Somogyi and Nelson method (Nelson, 1944).

## Production and Isolation of Xylanase II

Chainia sp. (NCL 82-5-1) was grown on the above medium, which contained bran as the major carbon source. After cultivation, as described, for 120 h, the cells and solid residues were removed from the culture broth by centrifugation at 700 x g for 20 min.

Enzyme Purification The following steps were carried out at 0 to 4°C, unless otherwise mentioned:

Step I: Alcohol precipitation

The culture filtrate (350 ml, 3,100 U) was concentrated by precipitating the enzyme with three volumes of chilled distilled ethanol at 4°C as described under Materials and Methods. The precipitate was recovered by centrifugation and dried under vacuum and stored at -10°C until further use. The recovery of xylanase activity was around 85-90%.

Step II: The alcohol precipitate (1.58 g, 2,800 U) of enzyme from Step I was dissolved in 50 ml of 50 mM, sodium phosphate buffer, pH 7.1. The undissolved solid particles from the precipitate were separated by centrifugation, and the clear enzyme solution was further purified on DEAE-cellulose in batch treatment.

DEAE-cellulose (batch treatment): DEAE-cellulose preactivited with 1 N NaOH and 1 N HCl was equilibrated with 50 mM, sodium phosphate buffer, pH 7.1. The solid DEAE-cellulose cake obtained by filtering 290 ml DEAE-cellulose slurry (containing 25 mg dry weight per ml) was suspended in 50 ml enzyme solution (2,800 U). This treatment was carried out under constant stirring for 30 min and the slurry was filtered through Whatman No. 1 filter paper. The filtrate was collected and DEAE-cellulose cake was resuspended twice in the same buffer each time using 25 ml buffer to recover the enzyme (875 U).

## Step III: DEAE-Fractogel-TSK-650 S ion-exchange chromatography

The concentrated enzyme solution from DEAE-cellulose treatment (Step II) was rechromatographed on DEAE-Fractogel-TSK-650 S column (2.0 x 90.0 cm), preequilibrated with 50 mM, sodium phosphate buffer, pH 7.1. The column was washed with the same buffer and 4 ml fractions were collected every 10 min. The yellowish pigment from the loaded sample was adsorbed on the top of the column. Fractions containing the unadsorbed xylanase were pooled (80 ml) and concentrated by ultrafiltration to 12 ml (765 U). The purified enzyme in this step gave a single peak for the xylanase activity, as shown in Fig. 1.

## Step IV: Separation of two xylanases by gel filtration

The concentrated enzyme solution from Step III was used for further purification by Sephadex G-50 column (2.5 to 110 cm). 50 mM, sodium phosphate buffer, pH 7.1, was used for loading as well as for elution of the enzyme. The xylanase activity and protein elution profiles are shown in Fig. 2. Fractions were collected at a flow rate as mentioned in Step III. The two xylanases designated as Xylanase I and Xylanase II (low molecular wt.) were resolved in 2 peaks at 135-170 ml (66 U) and 265-335 ml (265 U) respectively. The ratio of these two enzyme proteins was 30:70 and the ratio of their activities was 20:80. Specific activities of the purified Xylanase I and Xylanase II were 20.0 and 31.5 U/mg protein, respectively.

Purity of Xylanase I and II:

Purity of Xylanase II was further checked by analytical LKB isoelectric focusing procedure. The protein samples of Xylanase II were run along with the standard proteins in the pI range of 3.5 to 9.5. Samples of 20 to 30 μg were loaded on a polyacrylamide slab gel, electrophoresis was carried out for 8 h. The Xylanase II preparation showed a single protein band in disc gel electrophoresis at pH 4.3, indicating its homogeneity.

Table A summarizes the purification procedure, recovery and specific activity of Xylanase II at various stages.

TABLE A

| PURIFICATION OF XYLANASE II | | | | | | |
|---|---|---|---|---|---|---|
| Steps | Fractions | Volume (ml) | Total Protein (mg) | Total Activity xylanase (U) | Specific activity (U/mg) | Recovery (%) |
| | Crude filtrate | 350 | 3875 | 3100 | 0.8 | 100.0 |
| Step I | Alcohol precipitation | 50 | 1473 | 2800 | 1.9 | 85.0 |
| Step II | DEAE-cellulose | 110 | 134 | 875 | 6.5 | 28.2 |
| Step III | DEAE-Fractogel-TSK-650 S column | 80 | 37 | 765 | 20.6 | 24.7 |
| Step IV | Sephadex G-50 Xylanase II | 75 | 8/4 | 265 | 31.5 | 8.6 |

Additional Check on Purity

The Xylanase II prepared as described was subjected to cation exchange chromatography with a 20-mm i.d., 700-mm-long CM-Trisacryl column eluted with 0.8 mL/min of 0.025M, pH 4.5 sodium acetate buffer, with a 0-0.2M sodium chloride gradient. Only one protein peak exited the column; it contained all the Xylanase II activity (Fig. 2). This material was pure by SDS-PAGE and IEF conducted with pH 7 to pH 9 ampholytes.

## Properties and Kinetic Studies

Molecular weight: Molecular weight of Xylanase II was determined by four methods: (1) From the amino acids determined by hydrolysis and HPLC, adding one tryptophan residue found by sequencing the molecular weight is greater than 4087 daltons; (2) Using the amino acids found by sequencing, the molecular weight is greater than 4214 daltons; (3) By SDS-PAGE, with LKB protein molecular weight markers from 2,512 to 16,969 daltons, the molecular weight of underivatized Xylanase II is 6000 daltons. After pyridylethylation the moleclar weight 8000 daltons. By column chromatography with Sephadex G-50-50, using the same molecular weight standards, the molecular weight is 5000 daltons, (4) By column chromatography with Fractogel HW-405, the molecular weight was 9000 daltons.

Glycoprotein nature:

Two different methods were used to determine the glycoprotein nature of Xylanase II.

(a) Thymol:ethanol:$H_2SO_4$ Method: After polyacrylamide gel electrophoresis, the gels were immersed in a fixing solution containing iso-propanol:acetic acid:water (25:10:65) to fix the protein bands as well as to remove low molecular weight substances such as sucrose, glycerol and other impurities. The gels were further dipped for 1 h in 0.2% thymol prepared in the above fixing solution. The gels after rewashing with the fixing solution were transferred in a solution of concentrated $H_2SO_4$:
ethanol (80:20 v v) and kept for 3 h at 35°C. The glycoprotein of Xylanase II revealed as a red band on yellow background.

(b) Affinity chromatography on Concanavalin A Sepharose

4B: Concanavalin A Sepharose 4B has an affinity towards glycoproteins containing specifically α-D-mannopyranosyl and/or α-D-glucopyranosyl residues or internal 2-Q-D-mannopyranosyl residues. When Xylanase II was chromatographed on Concanavalin A Sepharose 4B column (0.6 x 8.0 cm), it was not adsorbed since the activity was detected in the first eluted fractions. This suggested that Xylanase II may contain sugar moieties other than α-D-mannopyranosyl or α-D-glucopyranosyl residues.

Amino acid composition:

Amino acid composition of Xylanase II was determined using Beckman automatic amino acid analyser. The results obtained are shown in Table B. It is assumed that methionine, isoleucine, leucine, phenylalanine and lysine, all with very similar molar concentrations, are represented by one amino acid residue each and that molar concentrations of histidine and cysteine are abnormally low, but also represent as one residue. The total number of residues is 42, plus one tryptophan residue not measured but inferred from amino acid sequencing.

Amino acid sequencing:

Amino acid sequence of Xylanase II (10 μg) was determined on an automatic gas phase sequenator. Before sequencing it was essential to know whether the N-terminal amino acid of Xylanase II was blocked or free. This was determined according to Chang (1983) using Dimethylaminoazobenzene isothiocyanate reagent. It was found that the N-terminal amino acid was free from any blockage.

The partial amino acid sequence of Xylanase II is shown in Diagram A, and amino acid composition from sequencing data is given in Table B. The sequencing data showed that N-terminal of the enzyme was alanine, while proline was present at the carboxyl end. The total amino acid residues detected by sequenator were 40.

## DIAGRAM A

### <u>Partial[(a)] Amino Acid[(c)] Sequence for Xylanase II</u>

1                        7           10
Ala-Thr-Thr-Ile-Thr-Thr-Asn-Gln-Thr-Gly-

11                                  20
Tyr-Asp-Gly-Met-Tyr-Tyr-Ser-Phe-Trp-Thr-

21                                  30
Asp-Gly-Gly-Gly-Ser-Val-Ser-Met-Thr-Leu-

31                     37          40
Asn-Gly-Gly-Gly-Thr-Tyr-Ser-Thr-Arg-Pro-

(Glu) [(b)]

---

(a) Sequence shown extends from N-terminal amino acid (Ala) to amino acid 40 (Pro) but is not completeto C-terminus.

(b) Amino acid 37 is probably serine but may be glutamic acid.

(c) Standard 3-letter symbols are used: Ala-alanine, Asn- Aspargine, Asp-aspartic acid, Gln-glutamine, Gly-glycine, Ile-isoleucine, Leu-leucine, Met-methionine, Phe-phenylalanine, Pro- proline, Ser-serine, Thr-threonine, Trp-tryptophan, Tyr-tyrosine, and Val-valine.

The amino acid sequence of Diagram A has been confirmed by repeated analysis for the 24 amino acid sequence from amino acid 7 (Asn) to amino acid 30 (Leu). The remaining portions of the molecule are believed to be generally correct, but there may be some amino acid variation due to inherent indefiniteness of amino acid analyzers. However, Diagram A should provide fingerprint-type identification for Xylanase II.

## TABLE B

| Amino Acid Compositions of Xylanase II | | | |
|---|---|---|---|
| Amino acids | Hydrolysis and HPLC | | Sequencing |
| | Mole % | Number of residues (to nearest non-zero integer) | Number of residues in partial sequence (1-40) |
| Aspartic acid | 10.55 | 4 | 4[a] |
| Threonine | 11.80 | 5 | 9 |
| Serine | 9.99 | 4 | 4 |
| Glutamic acid | 5.59 | 2 | 1[b] |
| Proline | 7.32 | 3 | 1 |
| Glycine | 16.75 | 7 | 8 |
| Alanine | 4.56 | 2 | 1 |
| Valine | 4.47 | 2 | 1 |
| Methionine | 2.77 | 1 | 2 |
| Isoleucine | 2.46 | 1 | 1 |
| Leucine | 2.59 | 1 | 1 |
| Tyrosine | 9.10 | 4 | 4 |
| Phenylalanine | 2.80 | 1 | 1 |
| Lysine | 2.37 | 1 | 0 |
| Histidine | 0.80 | 1 | 0 |
| Arginine | 5.23 | 2 | 1 |
| Cysteine | 0.85 | 1 | 0 |
| Tryptophan | (c) | (c) | 1 |

(a) 2 aspartic acid, 2 asparagine.
(b) Glutamine.
(c) Not determined.

nzymatic properties:

Purified Xylanase II was stable at pH 6.0 when stored a -15°C. No significant loss in activity was observed over a period of one year.

The enzyme showed no action on carboxymethyl cellulose, filter paper, p-nitrophenyl-$\beta$-D-xyloside and cellobiose. Optimum pH: Effect of pH on Xylanase II was tested using 50 mM, citrate buffer (pH 3.5 to 6.0) and 50 mM, sodium phosphate buffer (pH 6.5 to 8.0). Optimum pH for Xylanase II activity was found to be 6.0 at 50°C. Optimum temperature: Effect of temperature on Xylanase II was observed in the range of 4 to 80°C. Optimum temperature for Xylanase II activity on soluble xylan was found to be 60°C at pH 6.0.

## Deposit of Strain

On December 20, 1982, Chainia strain NCL-82-5-1 was placed on restricted, non-public deposit with The National Collection of Industrial Microorganisms, National Chemical Laboratory, Pune, India, under Accession No. 2980. A further deposit of Chainia strain NCL-82-5-1 has been made with The American Type Culture Collection, 12301 Parklawn Drive, Rockville, Maryland, 20852, U.S.A., under ATCC Accession No.53812

## REFERENCES

EP 0 353 342 A1

Iizuka and Kawaminami (1965), Agr. Biol. Chem., 29:520-524.
Kusakabe, Kawaguchi et al. (1977), Nippon Nogeikagaku Kaishi, 51:429-437.
Kusakabe, Yasui et al. (1977), Nippon Nogeika aku Kaishi, 51:439-448. Miller (1959), Anal. Chem., 31:426.
Nelson, J. Biol. Chem. (1944), 153:375-380.
Nakajima et al. (1984), J. Ferm. Technol., 62:269-276.
Nelson (1944), J. Biol. Chem., 153:375.
Srinivasan et al. (1983), Biotechnol. Lett. 5:611-614.
Srinivasan et al. (1984), Biotechnol. Lett. 6:715-718.
Vartak et al. (1984), Biotechnol. Lett. 6:493-494.

## Claims

1. An endo-xylanase, comprising a substantially pure glycoprotein having the following characteristics:
    (a) a molecular weight greater than 5,000 and less than 9,000; and
    (b) specific enzymatic activity for xylan with no appreciable enzymatic activity for cellulose.

2. The endo-xylanase of claim 1 purified to essential homogeneity.

3. The endo-xylanase of claim 1 or claim 2 which is further characterized by the following 24 amino acid sequence:
-Asn-Gln-Thr-Gly-Tyr-Asp-Gly-Met-Tyr-Tyr-Ser-Phe-Trp-Thr-Asp-Gly-Gly-Gly-Ser-Val-Ser-Met-Thr-Leu-
wherein Asn, Asp, Gln, Gly, Leu, Met, Phe, Ser, Thr, Trp, Tyr and Val, respectively, are asparagine, aspartic acid, glutamine, glycine, leucine, methionine, phenylalanine, serine, threonine, tryptophan, tyrosine, and valine.

4. An endo-xylanase, comprising a homogeneous preparation of the glycoprotein having the following characteristics:
    (a) a molecular weight of 6000 ± 1000;
    (b) specific activity for converting xylan to xylooligosaccharides; and
    (c) containing the amino acid sequence represented by:
-Asn-Gln-Thr-Gly-Tyr-Asp-Gly-Met-Tyr-Tyr-Ser-Phe-Trp-Thr-Asp-Gly-Gly-Gly-Ser-Val-Ser-Met-Thr-Leu-
wherein Asn, Asp, Gln, Gly, Leu, Met, Phe, Ser, Thr, Trp, Tyr, and Val, respectively, are asparagine, aspartic acid, glutamine, glycine, leucine, methionine, phenylalanine, serine, threonine, tryptophan, tyrosine, and valine.

FIG. 1

Xylanases

ABSORBANCE AT 280 nm

ELUTION VOLUME (mL)

ACTIVITY (U/mL)

EP 0 353 342 A1

FIG. 2

Xylanase I

Xylanase II

EP 0 353 342 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | DISSERTATION ABSTRACTS INTERNATIONAL, vol. 48, no. 7, January 1988, pages 2044-B - 2045-B, Ann Arbor, Michigan, US; M.M. MEAGHER et al.: "Characterization, kinetics, and subsite mapping of aspergillus niger glucoamylases I and II, and partial purification and characterization of a Chainia endo-xylanase" * Abstract * | 1-4 | C 12 N 9/24 // (C 12 N 9/24 C 12 R 1:645) |
| A,D | BIOTECHNOLOGY LETTERS, vol. 5, 1983, pages 611-614, Kew, GB; M.C. SRINIVASAN et al.: "High activity extracellular glucose (xylose) isomerase from a chainia species" | | |
| A,D | BIOTECHNOLOGY LETTERS, vol. 6, 1984, pages 715-718, Kew, GB; M.C. SRINIVASAN et al.: "Studies on xylan degrading enzyme from chainia" | | TECHNICAL FIELDS SEARCHED (Int. Cl.4) C 12 N |

The present search report has been drawn up for all claims

| Place of search | Date of completion of the search | Examiner |
|---|---|---|
| THE HAGUE | 20-10-1989 | VAN PUTTEN A.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)